# EUROPÄISCHE PATENTSCHRIFT

(11) **EP 3 175 784 B1**
(45) Veröffentlichungstag und Bekanntmachung des Hinweises auf die Patenterteilung: **27.03.2019**
(21) Anmeldenummer: 16206262.4
(22) Anmeldetag: 29.10.2013
(51) Int. Cl.: A61B 5/00, A61N 1/375, A61B 5/0478, A61B 5/0492

(54) **MIKROELEKTRODENARRAY**
MICRO-ELECTRODE ARRAY
RÉSEAU DE MICRO-ÉLECTRODES

(30) Priorität: 30.10.2012 DE 102012110358
(43) Veröffentlichungstag der Anmeldung: 07.06.2017
(62) Teilanmeldung aus: 13785435.2
(73) Patentinhaber: Leibniz-Institut für Neurobiologie Magdeburg, 39118 Magdeburg (DE); Otto-von-Guericke-Universität Magdeburg, 39106 Magdeburg (DE)
(72) Erfinder: Schmidt, Bertram, 78052 Villingen-Schwenningen (DE); Ohl, Frank, 39171 Osterweddingen (DE); Lippert, Michael, 39104 Magdeburg (DE); Deckert, Martin, 39104 Magdeburg (DE); Hirsch, Sören, 14789 Wusterwitz (DE)
(74) Vertreter: Gramm, Lins & Partner Patent- und Rechtsanwälte PartGmbB

(56) Entgegenhaltungen:
- WO-A2-2011/057276
- US-A1- 2005 203 366
- US-A1- 2007 100 398
- US-A1- 2013 144 365

## Beschreibung

Die Erfindung betrifft ein Mikroelektrodenarray mit einer Vielzahl von Elektroden zur elektrischen Messung von Hirnströmen gemäß dem Anspruch 1.

Es gibt bereits Ansätze zur Miniaturisierung von Elektroden zur Messung von Hirnströmen insbesondere in Form von sogenannten Mikroelektrodenarrays. Als Array bezeichnet man dabei eine Anordnung einer Vielzahl von Elektroden, die regelmäßig oder unregelmäßig angeordnet sein können, z.B. in einer Matrixanordnung. Mit solchen Elektroden bzw. solchen Mikroelektrodenarrays können elektrische Signale des Gehirns eines Lebewesens aufgenommen werden, wobei sowohl auf der Hirnoberfläche als auch innerhalb des Hirns Messungen möglich sind. Insbesondere kann mit solchen Mikroelektrodenarrays ein Elektrokortikogramm (ECoG) aufgenommen werden. Ein solches Mikroelektrodenarray ist in Dokument WO 2011/057276 offenbart.

Es gibt außerdem Entwicklungen im Bereich der sogenannten "Optogenetik", durch Licht Neurone im Hirn zu stimulieren, sofern diese bestimmte lichtsensitive Kanalproteine exprimieren. Typischerweise wird das für die Stimulation erforderliche Licht von außen durch Glasfasern oder an den Schädel montierte Leuchtdioden (LEDs) ins Hirn eingestrahlt.

Es ergeben sich hierbei mehrere Probleme, nämlich dass die Implantation der Lichtquelle und des Mikroelektrodenarrays räumlich viel Platz beansprucht, die Versuchsobjekte mit einer Glasfaserleitung an externe Technik angebunden sind und die genaue räumliche Ausrichtung von Lichtquelle und den einzelnen Elektroden zueinander schwierig ist. Der gesamte Implantationsvorgang ist durch die Notwendigkeit mehrerer Schritte und Technologien relativ kompliziert.

Der Erfindung liegt die Aufgabe zugrunde, dies zu vereinfachen.

Die Aufgabe wird durch ein Mikroelektrodenarray gemäß Anspruch 1 gelöst. Ein Vorteil der Erfindung ist es, dass die elektrische Lichtquelle bzw. die elektrischen Lichtquellen in das Mikroelektrodenarray integriert sind und somit nicht als separate Teile positioniert und implantiert werden müssen. Auch die elektrische Kontaktierung wird vereinfacht, da gemeinsame Leitungen, z.B. in Form eines Flachbandkabels, verwendet werden können. Ein weiterer Vorteil der Erfindung besteht darin, dass die elektrische Lichtquelle bzw. die elektrischen Lichtquellen in Bezug zu den Elektroden räumlich fixiert und exakt positioniert sind, wobei die exakte Positionierung bereits durch die Herstellung des Mikroelektrodenarrays vorgegeben ist. Daher ist das erfindungsgemäße Mikroelektrodenarray wesentlich anwendungsfreundlicher als bekannte Einrichtungen. Durch die Erfindung wird es möglich, kortikale Neuronen mit Hilfe von einer oder mehreren integrierten elektrischen Lichtquellen räumlich präzise und strukturiert optisch zu stimulieren und gleichzeitig die dadurch ausgelösten und spontanen Hirnströme elektrophysiologisch durch die mikrostrukturierten Elektroden abzuleiten.

Ein weiterer Vorteil besteht darin, dass die Möglichkeit besteht, Hirnbereiche mit verschiedenen räumlichen Mustern zu stimulieren, beispielsweise um topographische Beziehungen im Hirn auszumessen und zu stimulieren, z.B. für die Tonotopie, Somatotopie, Retinotopie.

Gemäß einer vorteilhaften Weiterbildung der Erfindung weist das Mikroelektrodenarray ein folienartiges, dünnes Substrat auf. Das Substrat kann insbesondere ein flexibles Substrat sein. Geeignete Materialien zur Herstellung des Substrats sind z.B. Folien aus Polyimid, Parylen, Polydimethylsiloxan (PDMS) oder Polyurethan. Es ist insbesondere vorteilhaft, ein optisch hinreichend transparentes Material, z.B. ein Folienmaterial, für das Substrat zu verwenden, so dass das von den elektrischen Lichtquellen abgegebene Licht das Substrat durchdringen kann. Das Substrat kann insbesondere mehrschichtig aus denselben oder verschiedenen Materialien aufgebaut sein, z.B. nach Art einer Sandwichstruktur.

Gemäß einer vorteilhaften Weiterbildung der Erfindung sind die Elektroden über die flächige Ausdehnung des Substrats verteilt an der Oberfläche des Substrats oder in dem Substrat angeordnet. Die Elektroden können z.B. als elektrisch leitende Beschichtung, z.B. als Metall-Beschichtung, auf der Oberfläche des Substrats oder als Metallschicht im Substrat ausgebildet sein. Gemäß einer vorteilhaften Weiterbildung der Erfindung sind eine Vielzahl von elektrischen Lichtquellen der Stimulationseinheit über die flächige Ausdehnung des Substrats verteilt an der Oberfläche des Substrats oder in dem Substrat angeordnet.

Die elektrischen Lichtquellen können insbesondere als Leuchtdioden ausgebildet sein, wobei anorganische Leuchtdioden oder organische Leuchtdioden (OLEDs) Anwendung finden können, auch in Kombination miteinander. Des Weiteren kann die elektrische Lichtquelle bzw. können die elektrischen Lichtquellen als phosphoreszierende organische Leuchtdioden (PHOLEDs) in den Aufbau des Mikroelektrodenarrays integriert sein oder in Kombination mit den zuvor genannten Lichtquellen Verwendung finden. Der verbesserte Wirkungsgrad der PHOLEDs im Vergleich zu anderen Lichtquellen ist vorteilhaft, da bei gleicher Lichtintensität eine geringere Wärmeentwicklung induziert und das Risiko einer Gewebeschädigung bzw. Schädigung von Hirnbereichen durch Erwärmung reduziert wird.

Gemäß einer vorteilhaften Weiterbildung der Erfindung ist vorgesehen, dass die elektrische Lichtquelle bzw. die elektrischen Lichtquellen nach einem festen vorgegebenen Schema relativ zu den Elektroden angeordnet und über die flächige Ausdehnung des Substrats verteilt sind. Hierdurch ist eine fest vorgegebene Zuordnung zwischen den Elektroden und den elektrischen Lichtquellen vorgegeben, so dass die über die Elektroden aufgenommene Stimulation der Neuronen mittels der elektrischen Lichtquellen in einzelnen Stimulationssignalen eindeutig zugeordnet werden kann, so dass eine eindeutige Korrelation bestimmt werden kann.

Gemäß einer vorteilhaften Weiterbildung der Erfindung sind auf der Oberfläche des Substrats oder in dem Substrat elektrisch leitende Strukturen gebildet, die die Elektroden, elektrische Verbindungsleitungen zu den Elektroden und/oder elektrische Verbindungsleitungen zu der elektrischen Lichtquelle bzw. den elektrischen Lichtquellen bilden. Dies hat den Vorteil, dass die elektrisch leitenden Strukturen, z.B. in Form von einer oder mehreren Metallschichten, in dem Substrat direkt eingebettet sein können oder darauf aufgebracht sein können, z.B. aufgedampft sein können.

Die elektrisch leitenden Strukturen können z.B. in Form einer oder mehrerer Metallisierungsebenen im Substrat bereit gestellt werden. Die Metallisierungsebene bzw. die Metallisierungsebenen, die Elektroden und/oder Verbindungsleitungen bildet, kann direkt zur Stromversorgung der Lichtquellen benutzt werden. Es kann auch eine zusätzliche gegebenenfalls dickere und/oder strukturierte Metallschicht zur Stromversorgung in dem Mikroelektrodenarray vorgesehen werden.

Gemäß der Erfindung weist das Mikroelektrodenarray eine Sensorseite auf, die dazu eingerichtet ist, in Kontakt mit der Hirnoberfläche eines zu untersuchenden Lebewesens gebracht zu werden. Vorteilhaft können dabei eine, mehrere oder alle elektrischen Lichtquellen in größerem Abstand von der Sensorseite angeordnet sein als die Elektroden. Dies ermöglicht eine günstige Unterbringung der Lichtquellen. Insbesondere ist es möglich, eine, mehrere oder alle elektrischen Lichtquellen auf der der Sensorseite abgewandten Oberfläche des Substrats anzuordnen. Des Weiteren kann durch die vorteilhafte Anordnung der elektrischen Lichtquelle bzw. der elektrischen Lichtquellen in relativem Abstand zur Sensorseite der Wärmeeintrag in die stimulierten Hirnbereiche verringert werden, um eine Gewebeschädigung zu unterbinden

Die elektrischen Lichtquellen können insbesondere als SMD-Bauelemente, als Dies oder als Dünnschichtelemente auf dem Substrat oder innerhalb des Substrats angeordnet sein.

Gemäß einer vorteilhaften Weiterbildung der Erfindung ist zumindest die Sensorseite des Mikroelektrodenarrays elektrisch und biologisch passiviert. Hierdurch werden unerwünschte gegenseitige Effekte mit dem Hirngewebe vermieden. Die Passivierung kann z.B. als Isolationsschicht ausgebildet sein.

Gemäß einer vorteilhaften Weiterbildung der Erfindung sind die Elektroden in das Material des Substrats integriert, z.B. darin eingebettet. Das Substrat weist dann an einer Sensorseite, die dazu eingerichtet ist, in Kontakt mit der Hirnoberfläche eines zu untersuchenden Lebewesens gebracht zu werden, zu den Elektroden führende Öffnungen auf. Hierdurch kann der elektrische Kontakt zu den Elektroden auch bei in das Material des Substrats integrierten Elektroden hergestellt werden. Die Integration der Elektroden in das Substrat hat den Vorteil, dass diese platzsparend darin angeordnet sind und besser vor Schädigungen geschützt sind.

Gemäß einer vorteilhaften Weiterbildung der Erfindung sind eine, mehrere oder alle Elektroden als ECoG-Elektroden ausgebildet.

Gemäß einer vorteilhaften Weiterbildung der Erfindung weist das Mikroelektrodenarray eine oder mehrere Stimulationselektroden zur Stimulation von Hirnbereichen mit elektrischen Signalen auf. Dies hat den Vorteil, dass zusätzlich eine Stimulation über elektrische Signale, die direkt galvanisch auf Hirnbereiche übertragen werden, möglich ist. Das Mikroelektrodenarray kann damit auch kombinierte Stimulationen mit optischen und elektrischen Signalen durchführen. Hierdurch werden die Anwendungsmöglichkeiten des Mikroelektrodenarrays erweitert.

Gemäß einer vorteilhaften Weiterbildung der Erfindung sind eine oder mehrere Elektroden zur elektrischen Messung von Hirnströmen zugleich Stimulationselektroden zur Stimulation von Hirnbereichen mit elektrischen Signalen. In diesem Fall besteht ein Vorteil darin, dass das Mikroelektrodenarray mit einer Vielzahl von Elektroden ausgebildet werden kann und diese wahlweise als Mess-Elektroden oder als Stimulationselektroden verwendet werden. Gemäß einer weiteren vorteilhaften Weiterbildung der Erfindung werden Elektroden zeitweise zur elektrischen Messung von Hirnströmen und zeitweise als Stimulationselektroden zur Stimulation von Hirnbereichen mit elektrischen Signalen eingesetzt. Eine entsprechende Steuerung der Funktion der jeweiligen Elektrode kann über eine Steuerelektronik erfolgen.

Gemäß einer vorteilhaften Weiterbildung der Erfindung sind in relativer Nähe zu einer, mehreren oder allen elektrischen Lichtquellen jeweils ein oder mehrere weitere elektrische und/oder elektronische Bauteile, insbesondere Sensorbauteile, angeordnet. Dies hat den Vorteil, dass über das oder die weiteren elektrischen bzw. elektronischen Bauteile je nach deren Ausführung weitere Möglichkeiten der Beeinflussung des Gewebes bzw. von Hirnbereichen möglich sind oder, wenn es sich um Sensorbauteile handelt, weitere Daten erfasst werden können. So kann ein weiteres elektrisches bzw. elektronisches Bauteil oder eine Gruppe solcher Bauteile jeweils einer elektrischen Lichtquelle zugeordnet sein und in deren Nähe angeordnet sein, z. B. neben der Lichtquelle oder um die Lichtquelle herum verteilt.

Gemäß einer vorteilhaften Weiterbildung der Erfindung sind in relativer Nähe zu einer, mehreren oder allen elektrischen Lichtquellen jeweils ein oder mehrere weitere elektrische und/oder elektronische Sensorbauteile, die jeweils ein elektrisches Sensorsignal abgeben, derart angeordnet, dass wenigstens eine von der elektrischen Lichtquelle oder den elektrischen Lichtquellen beeinflusste physikalische Größen durch das jeweilige Sensorbauteil erfassbar sind. Dies hat den Vorteil, dass durch solche Sensorbauteile von den Lichtquellen beeinflusste physikalische Größen überwacht werden können, wie z. B. das von der jeweiligen Lichtquelle abgegebene Licht, das direkt vor Ort ggf. unter Berücksichtigung von Reflektions- und/oder Absorptionseigenschaften des Hirngewebes erfasst werden kann. Auch andere von den elektrischen Lichtquellen beeinflusste physikalische Größen, wie z. B. die örtliche Temperatur, können erfasst werden. Die durch die Sensorbauteile erfassten Daten können z. B. auf einem Anzeigegerät (Display) angezeigt werden und damit visuell überwacht werden. Es kann auch eine automatische Überwachung auf Einhaltung zulässiger Grenzwerte durchgeführt werden, z. B. derart, dass bei Über- oder Unterschreiten bestimmter Grenzwerte automatisch ein Hinweissignal an den Benutzer erzeugt wird. Vorteilhaft ist es außerdem möglich, die erfassten Sensorsignale wiederum zur Beeinflussung der Lichtquellen bzw. des damit abgegebenen Lichts einzusetzen, so dass eine Steuerung der Lichtquellen in Abhängigkeit von den Sensorsignalen durchgeführt werden kann. Es kann auch ein vollständiger Regelkreis zur Regelung der elektrischen Lichtquellen in Abhängigkeit von Sensorsignalen der elektrischen und/oder elektronischen Sensorbauteile realisiert werden. Die Lichtquellen können dabei z. B. durch Variation elektrischer Parameter wie Strom und/oder Spannung gesteuert bzw. geregelt werden. Die Beeinflussung der elektrischen Parameter kann auch durch gepulste Signale erfolgen, deren Tastverhältnis variiert wird.

Gemäß einer vorteilhaften Weiterbildung der Erfindung weisen eine, mehrere oder alle elektrischen Lichtquellen der jeweiligen Lichtquelle zugeordnete ein oder mehrere Sensorbauteile auf, die jeweils ein elektrisches Sensorsignal abgeben, das der beeinflussten physikalischen Größe einer bestimmten Lichtquelle zuordenbar ist. Durch eine solche Zuordnungsmöglichkeit der von der Lichtquelle beeinflussten physikalischen Größe zu der Lichtquelle kann eine unabhängige, differenzierte Steuerung und/oder Regelung der jeweiligen einzelnen Lichtquelle gezielt durchgeführt werden, was insbesondere bei einer großen Anzahl elektrischer Lichtquellen vorteilhaft ist.

Gemäß einer vorteilhaften Weiterbildung der Erfindung sind einer, mehrere oder alle der Sensorbauteile als Temperatursensoren ausgebildet. Dies hat den Vorteil, dass die lokale Temperatur im Gewebe bzw. in Hirnbereichen gemessen werden kann. Insbesondere kann eine Messung der durch die Lichtquelle bzw. die Lichtquellen bedingten Temperaturerhöhung im Gewebe bzw. in den Hirnbereichen durchgeführt werden. In Verbindung mit der zuvor erwähnten Steuerung und/oder Regelung der Lichtquelle kann sichergestellt werden, dass die von den Lichtquellen verursachte Temperaturerhöhung in solchen Grenzen gehalten wird, dass eine Schädigung des Gewebes vermieden wird. Die Temperaturerhöhung kann somit regelungstechnisch auf zulässige Grenzwerte beschränkt werden.

Mit solchen Temperatursensoren kann auch eine Erwärmung von Stimulationselektroden, z.B. verursacht durch die Lichtquellen, erfasst werden.

Derartige Temperatursensoren können beispielsweise als Mäander ausgeführt sein und als Messprinzip die Temperaturabhängigkeit des elektrischen Widerstands der Metallisierung nutzen. Die Temperatursensoren können z. B. aus Platin hergestellt sein, wobei auch die Elektroden des Mikroelektrodenarrays aus Platin hergestellt sein können. Auch andere Ausführungsformen von Temperatursensoren sind einsetzbar.

Der Vorteil der Integration von Temperatursensoren basiert auf der zur Stimulation von Hirnbereichen mit optischen Signalen simultanen Temperaturmessungen in relativer Nähe zu der elektrischen Lichtquelle bzw. den elektrischen Lichtquellen, um das Risiko einer Gewebeschädigung bzw. Schädigung von Hirnbereichen durch unzulässige Erwärmung zu kontrollieren und/oder zu unterbinden.

Gemäß einer vorteilhaften Weiterbildung der Erfindung sind einer, mehrere oder alle der Sensorbauteile als lichtempfindliche Sensoren ausgebildet. Es können hierfür im Prinzip alle bekannten Arten lichtempfindlicher Sensoren eingesetzt werden, wie z. B. lichtempfindliche Widerstände oder Fotodioden. Besonders vorteilhaft sind anorganische und/oder organische Fotodioden, da sie gut in das Mikroelektrodenarray integrierbar sind. Der Einsatz solcher lichtempfindlichen Sensoren als weitere Sensorbauteile des Mikroelektrodenarrays hat den Vorteil, dass eine simultane Messung der Lichtintensität der stimulierenden optischen Signale möglich wird, welche die Korrelation der zur Stimulation kortikaler Neuronen bzw. von den Hirnbereichen aufgewendeten Lichtintensität zu der über die Elektroden aufgenommenen neuronalen Aktivität ermöglicht. Durch entsprechende computergestützte Auswertung der Signale der Elektroden sowie der Signale der lichtempfindlichen Sensoren können korrigierte Elektrodensignale bestimmt werden, die um unerwünschte Nebeneffekte wie z. B. Abschattungen von Lichtquellen oder Reflektionen des abgegebenen Lichts bereinigt sind. Gleichzeitig können die Absorption sowie die Streuung der stimulierenden optischen Signale durch kortikales Gewebe bestimmt werden. In Verbindung mit der zuvor erwähnten Steuerung bzw. Regelung der elektrischen Lichtquellen in Abhängigkeit von den Sensorsignalen kann auch eine automatische Korrekturfunktion der Lichtabgabe der Lichtquellen an gewünschte Lichtabgabewerte durchgeführt werden, indem die Sensorsignale daraufhin geprüft werden, ob die gewünschten Lichtabgabewerte der Lichtquellen erreicht werden, und falls dies nicht der Fall ist, korrigierend durch Steuerung bzw. Regelung der elektrischen Lichtquellen eingegriffen werden.

Die genannten Steuerung- bzw. Regelungsfunktionen können vorteilhaft durch eine elektronische Steuereinheit realisiert werden, die die Steuer- bzw. Regelfunktionen hardwaremäßig und/oder durch Softwaresteuerung durchführt, z. B. mit einem Mikroprozessor oder Mikrocontroller.

Gemäß einer vorteilhaften Weiterbildung der Erfindung sind die weiteren elektrischen und/oder elektronischen Bauteile nach einem festen, vorgegebenen Schema relativ zu den Lichtquellen angeordnet und über die flächige Ausdehnung des Substrats verteilt an der Oberfläche des Substrats oder in dem Substrat angeordnet. Des ermöglicht z. B. eine flächendeckende sensorische Erfassung von Größen über das gesamte Mikroelektrodenarray.

Gemäß einer vorteilhaften Weiterbildung der Erfindung sind auf der Oberfläche des Substrats oder in dem Substrat elektrisch leitende Strukturen gebildet, die elektrische Verbindungsleitungen zu dem weiteren elektrischen und/oder elektronischen Bauteil oder zu den weiteren elektrischen und/oder elektronischen Bauteilen bilden. Auf diese Weise können z. B. die Temperatursensoren und/oder die lichtempfindlichen Sensoren ohne externe Leitungen elektrisch kontaktiert werden. Die elektrischen Verbindungsleitungen können z. B. in gleicher Weise realisiert werden wie bei den elektrischen Lichtquellen.

Eine direkte Einbettung von elektrischen Verbindungsleitungen in dem Substrat kann z. B. durch Abscheidung metallischer Werkstoffe, z. B. durch Kathodenzerstäubung, durchgeführt werden. Eine solche Einbettung der elektrischen Verbindungsleitungen auf dem Substrat erhöht den Integrationsgrad und erlaubt damit eine erweiterte Funktionalität des Mikroelektrodenarrays, z. B. zur Temperaturmessung und Messung der Lichtintensität, ohne bauliche Vergrößerung.

Die elektrisch leitenden Strukturen, die z. B. als Metallisierungsebenen ausgebildet sind, können somit z. B. direkt zur Stromversorgung des Temperatursensors bzw. der Temperatursensoren und/oder des lichtempfindlichen Sensors bzw. der lichtempfindlichen Sensoren genutzt werden. Es ist dabei vorteilhaft, die Anzahl funktionsintegrierender Metallisierungsebenen nicht zu groß zu wählen, z. B. nur eine Metallisierungsebene zu verwenden. Hierdurch kann die erreichbare mechanische Flexibilität bei geringer Steifigkeit des Mikroelektrodenarrays verbessert werden, wodurch zugleich eine verbesserte Anpassungsfähigkeit des Mikroelektrodenarrays an die Topografie der Hirnoberfläche ermöglicht wird.

Gemäß einer vorteilhaften Weiterbildung der Erfindung sind eines, mehrere oder alle weiteren elektrischen und/oder elektronischen Bauteile als SMD-Bauteile, als Dies oder als Dünnschichtelemente auf dem Substrat oder innerhalb des Substrats angeordnet. Dies hat den Vorteil, dass die elektrischen bzw. elektronischen Bauteile platzsparend an oder in dem Substrat angeordnet sind und besser vor Schädigungen geschützt werden können. Die Annäherung eines, mehrerer oder aller elektrischen und/oder elektronischen Bauteile, insbesondere der Sensorbauteile wie der Temperatursensoren, im Substrat an die Sensorseite erlaubt die Erfassung von physikalischen Größen, insbesondere eine Temperaturmessung, die einen hohen Korrelationsgrad mit tatsächlichen physikalischen Größen stimulierter Hirnbereich aufweist.

Gemäß einer vorteilhaften Weiterbildung der Erfindung weist das Mikroelektrodenarray eine Empfangsvorrichtung zum drahtlosen Empfang von auf das Mikroelektrodenarray abgestrahlten elektromagnetischen Wellen auf. Das Mikroelektrodenarray ist mit der für seinen Betrieb notwendigen elektrischen Energie über die Empfangsvorrichtung drahtlos versorgbar und/oder ein Akkumulator des Mikroelektrodenarrays ist über die Empfangsvorrichtung drahtlos aufladbar. Die Empfangsvorrichtung kann z. B. als auf dem Substrat oder innerhalb des Substrats vorgesehene Spule ausgebildet sein, die z. B. aus dem Material der elektrisch leitenden Strukturen gebildet ist, aus denen die bereits erwähnten elektrischen Verbindungsleitungen zu den Lichtquellen oder den sonstigen elektrischen und/oder elektronischen Bauteilen gebildet sein können. Dies hat den Vorteil, dass keine Kabelverbindung zur Versorgung des Mikroelektrodenarrays mit elektrischer Energie erforderlich ist. Stattdessen kann z. B. eine in eine flexible Matte eingebettete Energiesendespule von außen auf die Hautoberfläche oder das Haar eines Patienten aufgelegt werden und von dieser über einen Hochfrequenzsignal das Mikroelektrodenarray über die Empfangsvorrichtung drahtlos mit elektrischer Energie versorgt werden. Ein ggf. in dem Mikroelektrodenarray vorhandener Akkumulator kann dadurch auch aufgeladen werden. Es kann z. B. ein Akkumulator vom Lithiumpolymer-Typ in Form einer flachen Schicht in dem Mikroelektrodenarray vorgesehen sein.

Gemäß einer vorteilhaften Weiterbildung der Erfindung weist das Mikroelektrodenarray eine drahtlos arbeitende Signalübertragungseinrichtung auf, die zum drahtlosen Übertragen von Signalen, insbesondere in Form von Daten, von dem Mikroelektrodenarray zu einer Signalempfangseinrichtung und/oder zum drahtlosen Übertragen von Signalen, insbesondere in Form von Daten, von einer Signalsendeeinrichtung zu dem Mikroelektrodenarray eingerichtet ist. Dies hat den Vorteil, dass keine Kabelverbindungen für die Signalübertragung bzw. die Datenübertragung erforderlich sind. Die Signalübertragungseinrichtung kann z. B. nach einem der bekannten Standards ausgebildet sein, z. B. als Bluetooth-Signalübertragungseinrichtung. Die Verwendung solcher kabellosen Verbindungen erlaubt die Senkung des Inflammationsrisikos nach erfolgter Implantation des Mikroelektrodenarrays. Der Vorteil beruht auf dem Wegfall von Kabeldurchführungen, wie z. B. Flachbandkabel, durch den Skalp. Insbesondere kann hierdurch auch das Infektionsrisiko reduziert werden. Die erforderlichen elektronischen Komponenten, z. B. Verstärker, Impedanzwandler, Filter, Multiplexer, Analog/Digital-Wandler, Energiespeicher können z. B. in einer anwendungsspezifischen integrierten Schaltung (ASIC) ganz oder teilweise zusammengeführt sein.

Gemäß der Erfindung sind eine, mehrere oder alle Elektroden in einer jeweils erhabenen Säulenstruktur integriert, welche die einzelnen Elektroden auf der Sensorseite von der Substratebene beabstandet. Dies hat den Vorteil, dass die Elektroden auf der Sensorseite etwas aus dem Substrat herausstehen können und damit besser bzw. dichter an zu sensierende Positionen an der Hirnoberfläche herangeführt werden können. Ferner kann hierdurch der Abstand zwischen den elektrischen Lichtquellen und der Hirnoberfläche vergrößert werden, so dass der Wärmeeintrag in die Hirnoberfläche reduziert wird. Die mikrotechnologische Fabrikation der Säulenstrukturen kann z. B. lithographische Maskierung und nasschemische Strukturierung einer metallischen Hartmaske erfolgen, wobei die Strukturübertragung in das Substrat beispielsweise mit Hilfe eines Trockenätzprozesses realisiert wird. Die Nutzung einer dickeren Substratfolie und/oder die sukzessive Abscheidung mehrerer Substratdünnschichten erlaubt die Herstellung von Säulenstrukturen mit variierenden Abmessungen. Kapillare Blutgefäße und biologische Strukturen auf der Hirnoberfläche, die die Annäherung planarer Mikroelektrodenarrays an die Hirnoberfläche limitieren, können durch derartige erhabene Säulenstrukturen überwunden werden. Das flexible Substratmaterial erlaubt dabei ein lokales Umgehen biologischer Strukturen. Der Vorteil derartiger Elektrodenstrukturen basiert demzufolge auf der Annäherung der Elektroden an die Hirnoberfläche, ohne diese zu penetrieren, und resultiert in einer verbesserten räumlichen Auflösung von Ableitung und Stimulation. Ein weiterer Vorteil ist, dass die dreidimensionalen Säulenstrukturen das Verrutschen implantierter Mikroelektrodenarrays unterbinden.

Gemäß einer vorteilhaften Weiterbildung der Erfindung sind eine, mehrere oder alle Säulenstrukturen aus dem Substratmaterial gefertigt. Dies erlaubt einen effizienten Herstellungsprozess des Mikroelektrodenarrays. Ferner ist die biologische Verträglichkeit weiterhin gegeben, da keine weiteren Materialien erforderlich sind.

Gemäß einer vorteilhaften Weiterbildung der Erfindung weisen eine, mehrere oder alle Säulenstrukturen eine jeweils umlaufende Dichtlippe auf, die die aktive Elektrodenfläche der in die Säulenstruktur integrierten Elektrode lateral begrenzt. Hierbei kann das Substrat wiederum an der Sensorseite zu den Elektroden führende Öffnungen aufweisen, die die aktiven Elektrodenflächen freigeben. Die umlaufenden Dichtlippen trennen die Elektrodenflächen von der umgebenden Zerebrospinalflüssigkeit, verhindern Ausgleichsströme zwischen den Elektroden und stellen den direkten Kontakt zur Hirnoberfläche her. Dies gewährleistet eine höchstmögliche räumliche Auflösung und beste Signal-Rausch-Abstände derartiger nicht penetrierender Mikroelektrodenarrays.

Gemäß einer vorteilhaften Weiterbildung der Erfindung sind Durchgangsöffnungen im Substrat des Mikroelektrodenarrays angeordnet, die dazu geeignet sind die Diffusion von pharmakologischen Substanzen ins Gewebe zu erlauben und/oder eine oder mehrere penetrierende Tiefenelektroden in das Gewebe einzubringen. Die Durchgangsöffnungen sind vorteilhaft an Positionen angeordnet, an denen nicht gerade die Elektroden, die elektrischen Lichtquellen oder die anderen elektrischen und/oder elektronischen Bauteile des Mikroelektrodenarrays angeordnet sind. So können die Durchgangsöffnungen z. B. in der Nähe der Elektroden, der elektrischen Lichtquelle bzw. den elektrischen Lichtquellen, dem weiteren elektrischen und/oder elektronischen Bauteil bzw. den Bauteilen im Substrat angeordnet sein. Die Durchgangsöffnungen erlauben es z. B. pharmakologische Substanzen z. B. für optogenetische Anwendungen ins Gewebe einzubringen. Es können auch eine oder mehrere penetrierende Tiefenelektroden in das kortikale Gewebe eingebracht werden. Hierdurch werden die Anwendungsmöglichkeiten des Mikroelektrodenarrays grundlegend erweitert. Gleichzeitig bedingen die Durchgangsöffnungen Kapillareffekte, die das Ansaugen des Mikroelektrodenarrays an die Hirnoberfläche und die Verdrängung umgebender Zerebrospinalflüssigkeit vorteilhaft beeinflussen.

Gemäß einer vorteilhaften Weiterbildung der Erfindung sind mehrere oder alle Durchgangsöffnungen nach einem festen, vorgegebenen Muster über die flächige Ausdehnung des Substrats verteilt angeordnet.

Gemäß einer vorteilhaften Weiterbildung der Erfindung sind eine oder mehrere Tiefenelektroden oder wenigstens ein Tiefenelektrodenarray zur simultanen elektrischen Messung von Hirnströmen tieferer Hirnbereiche und/oder der elektrischen sowie optischen Stimulation von tieferen Hirnbereichen in die Durchgangsöffnungen eingeführt. Als Tiefenelektrodenarray wird eine Anordnung von Tiefenelektroden verstanden, die an einem gemeinsamen Träger an festen Positionen zueinander angeordnet sind. Hierdurch werden die Anwendungsmöglichkeiten des Mikroelektrodenarrays grundlegend erweitert. Dies hat den Vorteil, dass der Signalraum auf Grund der Tiefeninformationen vergrößert und entsprechende Korrelationen in Ableitung und Stimulation eruiert werden können.

Die eingangs genannte Aufgabe wird ferner gelöst durch eine Einrichtung mit einem Mikroelektrodenarray der zuvor erläuterten Art und wenigstens einer elektronischen Steuereinrichtung, wobei die elektronische Steuereinrichtung mit einem, mehreren oder allen elektrischen und/oder elektronischen Bauteilen des Mikroelektrodenarrays gekoppelt ist. Insbesondere kann die elektronische Steuereinrichtung mit den Elektroden des Mikroelektrodenarrays, den elektrischen Lichtquellen, weiteren elektrischen und/oder elektronischen Bauteilen und/oder den Tiefenelektroden gekoppelt sein. Die elektronische Steuereinrichtung kann ganz oder teilweise in das Mikroelektrodenarray integriert sein. Auf Grund der Baugröße heutiger elektronischer Steuereinrichtungen ist es vorteilhaft, zumindest einen Teil der elektronischen Steuereinrichtung separat vom Mikroelektrodenarray zu realisieren, z. B. in einem gesonderten Steuergerät, das die vom Mikroelektrodenarray abgegebenen Signale aufnimmt und auswertet und ggf. Steuersignale an die elektrischen Lichtquellen abgibt. Die Steuerung der Elektroden in Abhängigkeit von Sensorsignalen des Mikroelektrodenarrays, z. B. im Rahmen des erwähnten Regelkreises, kann lokal auf dem Mikroelektrodenarray durch einen dort integrierten Teil der elektronischen Steuereinrichtung realisiert werden, oder extern vom Mikroelektrodenarray im separaten Steuergerät.

Die elektronische Steuereinrichtung kann mit einem, mehreren oder allen elektrischen und/oder elektronischen Bauteilen des Mikroelektrodenarrays drahtgebunden und/oder drahtlos gekoppelt sein. Die drahtlose Kopplung hat den Vorteil, dass das Mikroelektrodenarray nach seiner Implantation einfacher handhabbar ist und der Patient geschont wird.

Gemäß einer vorteilhaften Weiterbildung der Erfindung ist die elektronische Steuereinrichtung mit wenigstens einer, mehreren oder allen elektrischen Lichtquellen gekoppelt und zur Steuerung der Lichtabgabe von einer, mehreren oder allen gekoppelten elektrischen Lichtquellen eingerichtet.

Ferner kann vorgesehen sein, dass die elektronische Steuereinrichtung zur Steuerung der Lichtabgabe von einer, mehreren oder allen gekoppelten elektrischen Lichtquellen in Abhängigkeit von elektrischen und/oder elektronischen Sensorbauteilen des Mikroelektrodenarrays, die jeweils ein elektrisches Sensorsignal in Abhängigkeit von wenigstens einer von der elektrischen Lichtquelle oder den elektrischen Lichtquellen beeinflussten physikalischen Größe, die durch das jeweilige Sensorbauteil erfasst ist, eingerichtet ist.

Das Mikroelektrodenarray und die Einrichtung mit einem Mikroelektrodenarray können z.B. für das Mapping von Gehirnfunktionen und/oder zur Analyse epileptogener Zonen verwendet werden. Auch eine Anwendung als Mensch-Maschine-Schnittstelle (HMI) bzw. Gehirn-Maschine-Schnittstelle (BMI) ist vorteilhaft.

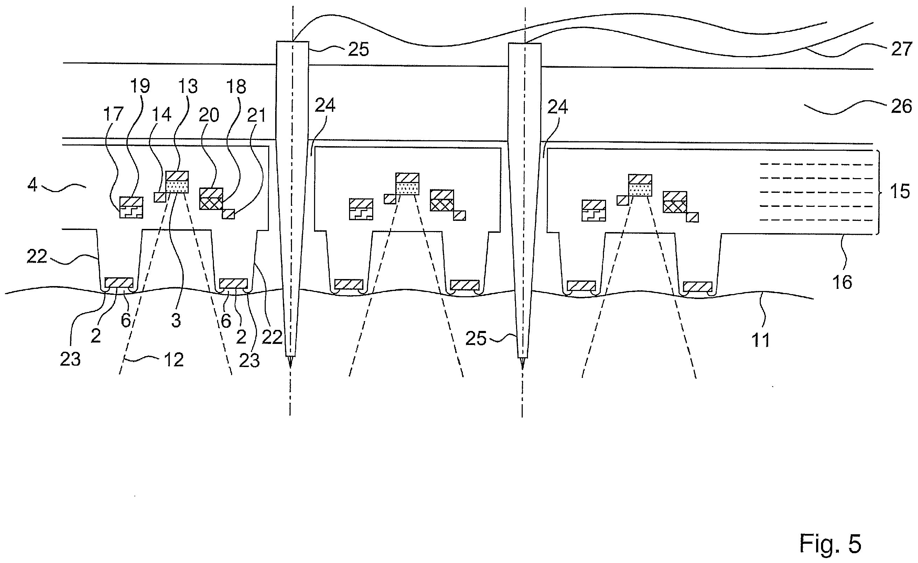

Die Erfindung wird nachfolgend anhand des Ausführungsbeispiels gemäß Figur 5 näher erläutert.

Die Figuren zeigen
- Fig. 1:: ein Mikroelektrodenarray in isometrischer Darstellung und
- Fig. 2, 3 und 5:: Ausführungsformen eines Mikroelektrodenarrays in seitlicher Schnitt darstellung und
- Fig. 4 und 6:: die Anwendung eines Mikroelektrodenarrays bei der Aufnahme eines Elektrokortikogrammes.

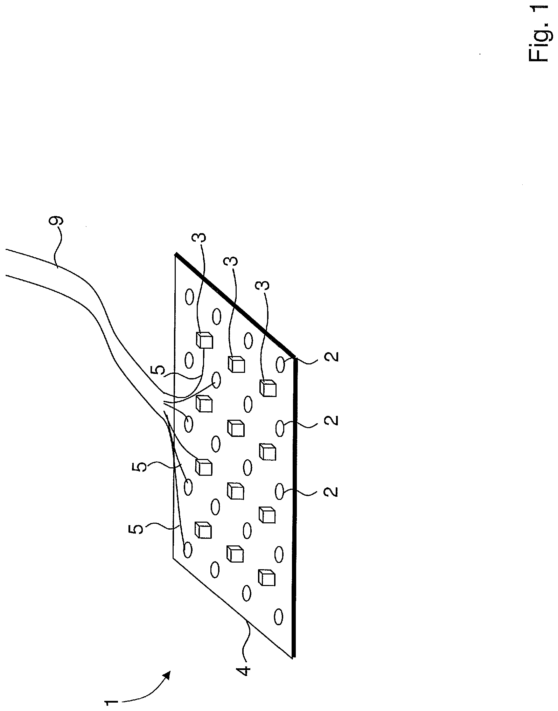

In den Figuren werden gleiche Bezugszeichen für einander entsprechende Elemente verwendet. Figur 5 zeigt das Ausführungsbeispiel der Erfindung. Die Fig. 1 zeigt ein Mikroelektrodenarray 1, das z.B. als Dünnschichtarray mit einem flexiblen, folienartigen dünnen Substrat 4 ausgebildet sein kann. An oder in dem Substrat 4 sind mehrere Elektroden 2, die jeweils durch Kreise dargestellt sind, und mehrere elektrische Lichtquellen 3 in Form von Leuchtdioden, die jeweils in Form von Quadern dargestellt sind, angeordnet. Die Elektroden 2 und die Leuchtdioden 3 sind über elektrische Leitungen 5, von denen aus Übersichtsgründen in der Fig. 1 nur einige Leitungen beispielhaft dargestellt sind, mit einem Verbindungskabel 9 verbunden. Das Verbindungskabel 9 kann z.B. als Flachbandkabel ausgebildet sein. Über das Verbindungskabel 9 werden die elektrischen Signale der Elektroden 2 zu einem Verstärker und einem Messsystem geleitet und zudem die Leuchtdioden 3 mit Strom versorgt.

Das Mikroelektrodenarray 1 kann hinsichtlich der Breite und Länge Maße im Millimeter- oder Zentimeterbereich haben und in unterschiedlichen Formen, die auch von der in Fig. 1 dargestellten Rechteckform abweichen können, ausgebildet sein.

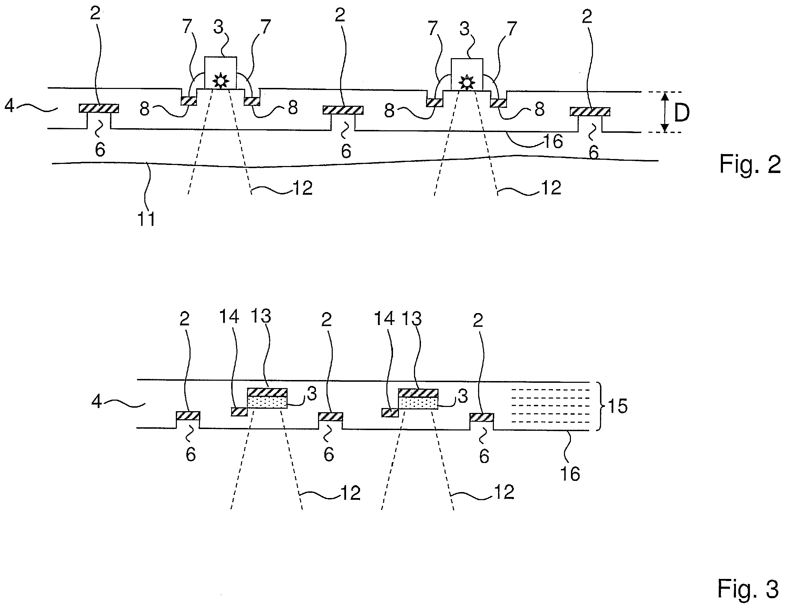

Die Fig. 2 zeigt eine Ausführungsform des Mikroelektrodenarrays 1 im Querschnitt. Die Dicke D des Substrats 4 ist relativ gering im Vergleich zur Breite und Länge. Die Dicke D kann insbesondere im Mikrometer-Bereich liegen. Das als dünne, flexible Folie z.B. aus Parylen, Polyimid, PDMS oder Polyurethan ausgebildete Substrat 4 weist darin eingebrachte Metallstrukturen 2, 8 auf. Die Metallstrukturen 2 bilden in mit Öffnungen 6 versehenen Substratbereichen die Elektroden 2. Die Öffnungen 6 weisen zu einer Sensorseite 16 des Substrats 4 hin, die dazu eingerichtet ist, in Kontakt mit der Hirnoberfläche 11 eines zu untersuchenden Lebewesens gebracht zu werden. Die Metallstrukturen 8 realisieren von den Elektroden 2 separate, d.h. elektrisch davon getrennte, sowie zum biologischen Gewebe bzw. Cortex 11 passivierte Stromversorgungsleitungen der Leuchtdioden 3. Die Leuchtdioden 3 sind auf der der Hirnoberfläche 11 abgewandten Seite des Substrats 4 auf dieses Substrat 4 aufgebracht oder in das Substrat 4 integriert, z. B. nach Art einer Sandwichstruktur. Die Leuchtdioden 3 sind entweder direkt oder mit elektrischen Verbindungen 7, z.B. in Form von Bonds, mit den Stromversorgungsleitungen 8 elektrisch verbunden. Die Leuchtdioden 3 strahlen ihr Licht 12 durch das optisch hinreichend transparente Substrat 4 in Richtung zur Hirnoberfläche 11 ab und stimulieren dadurch die darin vorhandenen Nervenzellen. Die Nervenzellen können z.B. mit Kanalrhodopsinen lichtsensitiv gemacht werden. Der Pfad des Lichts 12 kann dabei auch durch die Metallstrukturen 2, 8 oder durch zusätzliche Elemente, wie z.B. refraktive oder reflektive optische Elemente, die im Mikroelektrodenarray 1 vorhanden sind, beeinflusst werden.

Die Fig. 3 zeigt eine weitere Ausführungsform eines Mikroelektrodenarrays in Querschnittsdarstellung. Gemäß Fig. 3 sind die Leuchtdioden 3 in Form von in das Substrat 4 integrierten Dünnschicht-LEDs ausgebildet, insbesondere als organische LEDs. Das Substrat 4 kann in diesem Fall vorteilhaft als Mehrschichtstruktur 15 aus mehreren Lagen hergestellt sein. Die Leuchtdioden 3 sind dann direkt in die Mehrschichtstruktur 15 des Substrats 4 mikrostrukturiert eingebracht. Sie werden durch Metalllayer 13, 14 in der Mehrschichtstruktur 15 kontaktiert, um die Stromversorgung zu gewährleisten. Die Elektroden 2 sind wiederum über Öffnungen 6 zur Sensorseite 16 hin geöffnet. Alle anderen Strukturen sind elektrisch und biologisch passiviert.

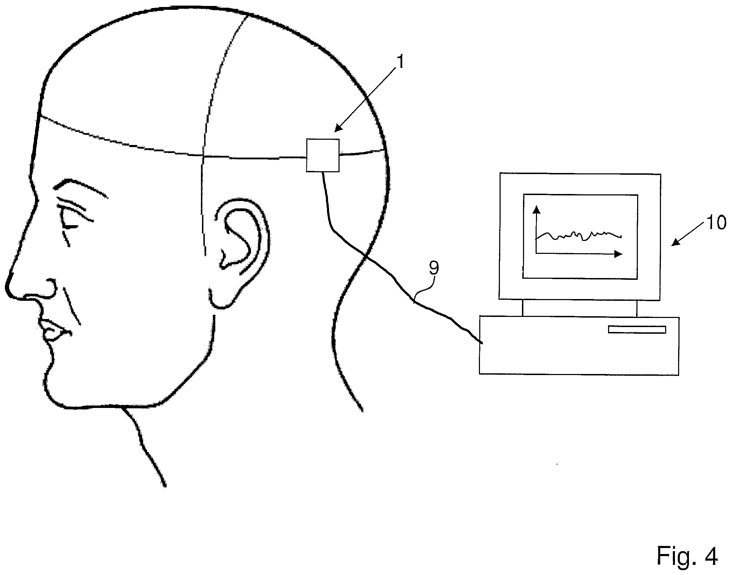

Die Fig. 4 zeigt eine Anwendung des erfindungsgemäßen Mikroelektrodenarrays 1 bei der Aufnahme eines Elektrokortikogramms eines Menschen. Das Mikroelektrodenarray 1 ist über das Verbindungskabel 9 mit einer elektronischen Einrichtung 10 verbunden, die insbesondere einen Verstärker für die Elektrodensignale und ein Datenerfassungssystem beinhaltet. Die aufgenommenen Daten können z.B. auf einem Bildschirm dargestellt werden.

Die Fig. 5 zeigt die Ausführungsform eines Mikroelektrodenarrays gemäß Anspruch 1 in Querschnittsdarstellung. Gemäß Fig. 5 sind die Elektroden 2 in erhabenen, aus dem Substratmaterial hergestellten Säulenstrukturen 22 zur Annäherung an die Hirnoberfläche 11 eingebettet und wiederum über Öffnungen 6 zur Sensorseite 16 hin geöffnet. Mikrostrukturierte, umlaufende Dichtlippen 23 begrenzen die aktiven Elektrodenflächen der Elektroden 2 lateral. Die Leuchtdioden 3 sind in Form von in das Substrat 4 integrierten Dünnschicht-LEDs ausgebildet, insbesondere als phosphoreszierende organische Leuchtdioden, und sind direkt in die Mehrschichtstruktur 15 des Substrats 4 mikrostrukturiert eingebracht. Sie werden durch die Metallstrukturen 13, 14 in der Mehrschichtstruktur 15 kontaktiert, um die Stromversorgung zu gewährleisten und strahlen ihr Licht 12 durch das optisch hinreichend transparente Substrat 4 in Richtung zur Hirnoberfläche 11 ab. Die Metallstrukturen 13, 14, 19, 20, 21 realisieren von den Elektroden 2 separate, d.h. elektrisch davon getrennte, sowie zum biologischen Gewebe bzw. Cortex 11 passivierte, elektrische Verbindungsleitungen. Dabei bilden die Metallstrukturen 19 die Stromversorgungsleitung der beispielsweise als Mäander ausgeführten Temperatursensoren 17 und die Metallstrukturen 20, 21 die elektrische Kontaktierung der Photodioden 18. Temperatursensoren 17 sowie Photodioden 18 sind im Substrat, z.B. nach Art einer Sandwichstruktur, eingebettet und somit elektrisch und biologisch passiviert. In dem Substrat 4 sind Durchgangsöffnungen 24 angeordnet, die dazu geeignet sind die Diffusion von pharmakologischen Substanzen ins Gewebe, für z.B. optogenetische Anwendungen, zu erlauben und/oder penetrierende Tiefenelektroden 25 in das kortikale Gewebe einzubringen. Die Tiefenelektroden 25 sind in einem angepassten Tiefenelektrodenarray 26 in definiertem Abstand ortsfest integriert, wobei die kabelgebundenen, z.B. als Flachbandkabel ausgeführten, elektrischen Verbindungsleitungen 27 auf der von der Sensorseite 16 abgewandten Seite weggeführt werden.

Die Fig. 5 zeigt nebeneinander drei der zuvor beschriebenen Anordnungen in dem Substrat 4, die gleich aufgebaut sind und von denen daher wegen der besseren Übersichtlichkeit nur eine Anordnung vollständig mit Bezugszeichen versehen wurde.

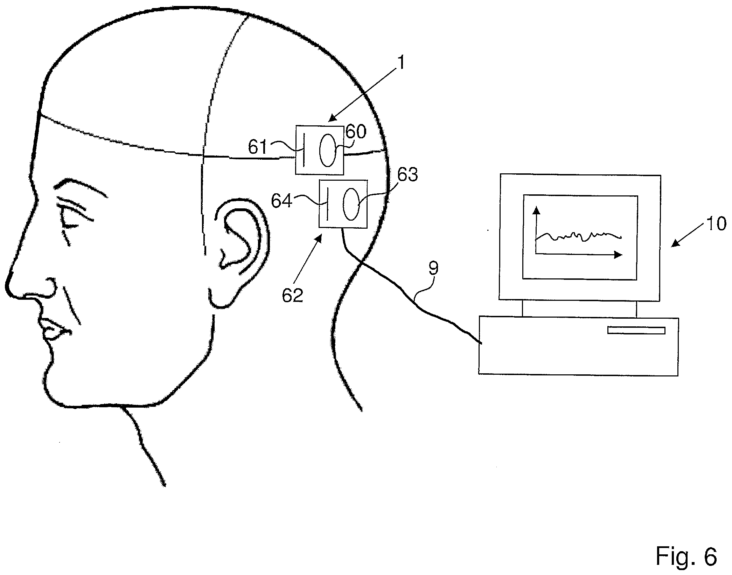

Die Fig. 6 zeigt eine Anwendung des erfindungsgemäßen Mikroelektrodenarrays 1 bei der Aufnahme eines Elektrokortikogramms eines Menschen. Im Unterschied zur Fig. 4 ist das Mikroelektrodenarray 1 drahtlos mit einer elektronischen Steuereinrichtung 10 verbunden, die insbesondere einen Verstärker für die Elektrodensignale und ein Datenerfassungssystem beinhaltet. Das z.B. unter dem Skalp des Patienten implantierte Mikroelektrodenarray 1 weist eine Energieempfangsspule 60 sowie eine Antenne 61 für die bidirektionale Datenübertragung zwischen dem Mikroelektrodenarray 1 und der elektronischen Steuereinrichtung 10 auf. Es ist auch möglich, die Energieempfangsspule 60 zugleich als Antenne zu verwenden, so dass keine separate Antenne 61 erforderlich ist.

Die elektronische Steuereinrichtung 10 ist hierfür über ein Kabel 9 mit einen Satellitenpad 62 verbunden, in dem eine Energiesendespule 63 und eine Antenne 64 angeordnet sind. Über die Energiesendespule 63 wird über ein Hochfrequenzsignal elektrisch Energie in die Energieempfangsspule 60 eingespeist, so dass das Mikroelektrodenarray 1 drahtlos mit der für seinen Betrieb erforderlichen elektrischen Energie versorgt wird. Über die Antennen 61, 63 erfolgt eine bidirektionale Datenkommunikation zwischen der elektronischen Steuereinrichtung 10 und dem Mikroelektrodenarray 1.

## Patentansprüche

1. Mikroelektrodenarray (1) zur Aufnahme eines Elektrokortigramms (ECoG) mit einem Substrat (4), mit einer Vielzahl von Elektroden (2) in Form von die Hirnoberfläche nicht penetrierenden ECoG-Elektroden zur elektrischen Messung von Hirnströmen und mit integrierter optischer Stimulationseinheit zur Stimulation von Hirnbereichen (11) mit optischen Signalen, wobei die Stimulationseinheit eine oder mehrere elektrische Lichtquellen (3) aufweist, wobei das Substrat (4) dazu eingerichtet ist, auf einer Sensorseite (16) in Kontakt mit der Hirnoberfläche (11) eines zu untersuchenden Lebewesens (11) gebracht zu werden, wobei eine, mehrere oder alle Elektroden (2) in einer jeweils erhabenen Säulenstruktur (22) integriert sind, welche eine aktive Elektrodenfläche der einzelnen Elektroden (2) auf der Sensorseite (16) von dem Substrat (4) beabstandet, **dadurch gekennzeichnet, dass** eine, mehrere oder alle Säulenstrukturen (19) eine jeweils umlaufende Dichtlippe (23) aufweisen, die die aktive Elektrodenfläche der in die Säulenstruktur (22) integrierten Elektrode (2) lateral begrenzt.

2. Mikroelektrodenarray nach Anspruch 1, **dadurch gekennzeichnet, dass** die elektrische Lichtquelle (3) bzw. die elektrischen Lichtquellen (3) nach einem festen, vorgegebenen Schema relativ zu den Elektroden (2) angeordnet und über die flächige Ausdehnung des Substrats (4) verteilt sind.

3. Mikroelektrodenarray nach einem der vorhergehenden Ansprüche,
**dadurch gekennzeichnet, dass** das Mikroelektrodenarray (1) eine oder mehrere Stimulationselektroden zur Stimulation von Hirnbereichen mit elektrischen Signalen aufweist.

4. Mikroelektrodenarray nach Anspruch 3, **dadurch gekennzeichnet, dass** eine oder mehrere Elektroden (2) zur elektrischen Messung von Hirnströmen zugleich Stimulationselektroden zur Stimulation von Hirnbereichen mit elektrischen Signalen sind.

5. Mikroelektrodenarray nach einem der vorhergehenden Ansprüche,
**dadurch gekennzeichnet, dass** das Mikroelektrodenarray (1) eine Empfangsvorrichtung zum drahtlosen Empfang von auf das Mikroelektrodenarray (1) abgestrahlten elektromagnetischen Wellen aufweist und das Mikroelektrodenarray (1) mit der für seinen Betrieb notwendigen elektrischen Energie über die Empfangsvorrichtung drahtlos versorgbar ist und/oder ein Akkumulator des Mikroelektrodenarrays (1) über die Empfangsvorrichtung drahtlos aufladbar ist.

6. Mikroelektrodenarray nach einem der vorhergehenden Ansprüche,
**dadurch gekennzeichnet, dass** das Mikroelektrodenarray (1) eine drahtlos arbeitende Signalübertragungseinrichtung aufweist, die zum drahtlosen Übertragen von Signalen, insbesondere in Form von Daten, von dem Mikroelektrodenarray (1) zu einer Signalempfangseinrichtung und/oder zum drahtlosen Übertragen von Signalen, insbesondere in Form von Daten, von einer Signalsendeeinrichtung zu dem Mikroelektrodenarray (1) eingerichtet ist.

7. Mikroelektrodenarray (1) nach einem der vorhergehenden Ansprüche,
**dadurch gekennzeichnet, dass** eine, mehrere oder alle Säulenstrukturen (19) aus dem Substratmaterial gefertigt sind.

8. Mikroelektrodenarray (1) nach einem der vorhergehenden Ansprüche,
**dadurch gekennzeichnet, dass** Durchgangsöffnungen (24) im Substrat (4) angeordnet sind, die dazu geeignet sind die Diffusion von pharmakologischen Substanzen ins Gewebe zu erlauben und/oder eine oder mehrere penetrierende Tiefenelektroden (25) in das Gewebe einzubringen.

9. Mikroelektrodenarray nach Anspruch 8, **dadurch gekennzeichnet, dass** mehrere oder alle Durchgangsöffnungen (24) nach einem festen, vorgegebenen Muster über die flächige Ausdehnung des Substrats (4) verteilt angeordnet sind.

10. Mikroelektrodenarray (1) nach Anspruch 8 oder 9, **dadurch gekennzeichnet, dass** eine oder mehrere Tiefenelektroden (25) oder wenigstens ein Tiefenelektrodenarray (26) zur simultanen elektrischen Messung von Hirnströmen tieferer Hirnbereiche und/oder der elektrischen sowie optischen Stimulation von tieferen Hirnbereichen in die Durchgangsöffnungen (24) eingeführt sind.

11. Einrichtung mit einem Mikroelektrodenarray nach einem der vorhergehenden Ansprüche und wenigstens einer elektronischen Steuereinrichtung, **dadurch gekennzeichnet, dass** die elektronische Steuereinrichtung mit einem, mehreren oder allen elektrischen und/oder elektronischen Bauteilen des Mikroelektrodenarrays (1) gekoppelt ist.

12. Einrichtung nach Anspruch 11, **dadurch gekennzeichnet, dass** die elektronische Steuereinrichtung mit einem, mehreren oder allen elektrischen und/oder elektronischen Bauteilen des Mikroelektrodenarrays (1) drahtgebunden und/oder drahtlos gekoppelt ist.

13. Einrichtung nach einem der Ansprüche 11 bis 12, **dadurch gekennzeichnet, dass** die elektronische Steuereinrichtung wenigstens mit einer, mehreren oder allen elektrischen Lichtquellen (3) gekoppelt ist und zur Steuerung der Lichtabgabe von einer, mehreren oder allen gekoppelten elektrischen Lichtquellen (3) eingerichtet ist.

14. Einrichtung nach Anspruch 12, **dadurch gekennzeichnet, dass** die elektronische Steuereinrichtung zur Steuerung der Lichtabgabe von einer, mehreren oder allen gekoppelten elektrischen Lichtquellen (3) in Abhängigkeit von elektrischen und/oder elektronischen Sensorbauteilen des Mikroelektrodenarrays (1), die jeweils ein elektrisches Sensorsignal in Abhängigkeit von wenigstens einer von der elektrischen Lichtquelle (3) oder den elektrischen Lichtquellen (3) beeinflussten physikalischen Größe, die durch das jeweilige Sensorbauteil erfasst ist, eingerichtet ist.

## Claims

1. Microelectrode array (1) for recording an electrocorticogram (ECoG) having a substrate (4), having a multiplicity of electrodes (2) in the form of ECoG electrodes that do not penetrate the surface of the brain for the purpose of electrically measuring brainwaves, and having an integrated optical stimulating unit for stimulating brain regions (11) with optical signals, wherein the stimulation unit comprises one or more electrical light sources (3), wherein the substrate (4) is configured, on a sensor side (16), to be brought into contact with the surface (11) of the brain of a living being (11) to be examined, wherein one, a plurality or all of the electrodes (2) are integrated in a respectively elevated column structure (22) which spaces apart an active electrode area of the individual electrodes (2) on the sensor side (16) from the substrate (4), **characterized in that** one, a plurality or all of the column structures (19) have a respectively circumferential sealing lip (23) which laterally delimits the active electrode area of the electrode (2) integrated in the column structure (22).

2. Microelectrode array according to Claim 1, **characterized in that** the electrical light source (3) or the electrical light sources (3) is/are arranged according to a fixed, predefined scheme relative to the electrodes (2) and distributed over the areal extent of the substrate (4).

3. The microelectrode array according to either of the preceding claims, **characterized in that** the microelectrode array (1) comprises one or more stimulation electrodes for stimulating brain regions with electrical signals.

4. Microelectrode array according to Claim 3, **characterized in that** one or more electrodes (2) for electrically measuring brainwaves are simultaneously stimulation electrodes for stimulating brain regions with electrical signals.

5. Microelectrode array according to any of the preceding claims, **characterized in that** the microelectrode array (1) comprises a receiving apparatus for wirelessly receiving electromagnetic waves radiated onto the microelectrode array (1), and the microelectrode array (1) is able to be supplied with the electrical energy necessary for its operation wirelessly via the receiving apparatus and/or a rechargeable battery of the microelectrode array (1) is chargeable wirelessly via the receiving apparatus.

6. Microelectrode array according to any of the preceding claims, **characterized in that** the microelectrode array (1) comprises a wirelessly operating signal transfer device configured for wirelessly transferring signals, in particular in the form of data, from the microelectrode array (1) to a signal receiving device and/or for wirelessly transferring signals, in particular in the form of data, from a signal transmitting device to the microelectrode array (1).

7. Microelectrode array (1) according to any of the preceding claims, **characterized in that** one, a plurality or all of the column structures (19) are produced from the substrate material.

8. Microelectrode array (1) according to any of the preceding claims, **characterized in that** through openings (24) are arranged in the substrate (4) and are suitable for allowing the diffusion of pharmacological substances into the tissue and/or for introducing one or more penetrating depth electrodes (25) into the tissue.

9. Microelectrode array according to Claim 8, **characterized in that** a plurality or all of the through openings (24) are arranged in a manner distributed according to a fixed, predefined pattern over the aerial extent of the substrate (4) .

10. Microelectrode array (1) according to Claim 8 or 9, **characterized in that** one or more depth electrodes (25) or at least one depth electrode array (26) for simultaneously electrically measuring brainwaves of deeper brain regions and/or electrically and optically stimulating deeper brain regions are inserted into the through openings (24).

11. Device comprising a microelectrode array according to any of the preceding claims and at least one electronic control device, **characterized in that** the electronic control device is coupled to one, a plurality or all of the electrical and/or electronic components of the microelectrode array (1).

12. Device according to Claim 11, **characterized in that** the electronic control device is coupled to one, a plurality or all of the electrical and/or electronic components of the microelectrode array (1) in a wired fashion and/or wirelessly.

13. Device according to either of Claims 11 to 12, **characterized in that** the electronic control device is coupled at least to one, a plurality or all of the electrical light sources (3) and is configured for controlling the emission of light from one, a plurality or all of the coupled electrical light sources (3).

14. Device according to Claim 12, **characterized in that** the electronic control device is configured for controlling the emission of light from one, a plurality or all of the coupled electrical light sources (3) in a manner dependent on electrical and/or electronic sensor components of the microelectrode array (1) which in each case an electrical sensor signal in a manner dependent on at least one physical variable which is influenced by the electrical light source (3) or the electrical light sources (3) and which is detected by the respective sensor component.

## Revendications

1. Réseau de micro-électrodes (1) destiné à acquérir un électrocortigramme (ECoG), comportant un substrat (4), une pluralité d'électrodes (2) sous la forme d'électrodes ECoG qui ne pénètrent pas à travers la surface cérébrale, pour mesurer électriquement des courants cérébraux, et comportant une unité de stimulation optique intégrée destinée à stimuler des régions cérébrales (11) par des signaux optiques, dans lequel l'unité de stimulation comprend une ou plusieurs sources lumineuses électriques (3), dans lequel le substrat (4) est conçu pour être amené, du côté d'un capteur (16), au contact de la surface cérébrale (11) d'un être vivant (11) à examiner, dans lequel l'une, plusieurs ou la totalité des électrodes (2) est/sont intégrée(s) à une structure de colonne surélevée respective (22) qui, du côté du capteur (16), sépare une surface d'électrode active des électrodes individuelles (2) du substrat (4), **caractérisé en ce que** l'une, plusieurs ou la totalité des structures de colonnes (19) présente/présentent un rebord d'étanchéité périphérique respectif (23) qui délimite latéralement la surface active de l'électrode (2) intégrée à la structure de colonne (22).

2. Réseau de micro-électrodes selon la revendication 1, **caractérisé en ce que** la source lumineuse électrique (3) ou les sources lumineuses électriques (3) est/sont disposée(s) selon un schéma fixe prédéterminé par rapport aux électrodes (2) et est/sont répartie(s) sur l'étendue plane du substrat (4).

3. Réseau de micro-électrodes selon l'une des revendications précédentes, **caractérisé en ce que** le réseau de micro-électrodes (1) comprend une ou plusieurs électrodes de stimulation destinées à stimuler des régions cérébrales par des signaux électriques.

4. Réseau de micro-électrodes selon la revendication 3, **caractérisé en ce qu'**une ou plusieurs électrodes (2) destinées à la mesure électrique de courants cérébraux sont en même temps des électrodes de stimulation destinées à la stimulation de régions cérébrales par des signaux électriques.

5. Réseau de micro-électrodes selon l'une des revendications précédentes, **caractérisé en ce que** le réseau de micro-électrodes (1) comprend un dispositif de réception destiné à recevoir sans fil des ondes électromagnétiques rayonnées vers le réseau de micro-électrodes (1) et **en ce que** le réseau de micro-électrodes (1) peut être alimenté sans fil avec l'énergie électrique nécessaire à son fonctionnement par l'intermédiaire du dispositif de réception et/ou **en ce qu'**un accumulateur du réseau de micro-électrodes (1) peut être chargé sans fil par l'intermédiaire du dispositif de réception.

6. Réseau de micro-électrodes selon l'une des revendications précédentes, **caractérisé en ce que** le réseau de micro-électrodes (1) comporte un dispositif de transmission de signaux sans fil qui est conçu pour la transmission sans fil de signaux, en particulier sous forme de données, depuis le réseau de micro-électrodes (1) vers un dispositif de réception de signaux et/ou pour la transmission sans fil de signaux, en particulier de données, depuis un dispositif d'émission de signaux vers le réseau de micro-électrodes (1).

7. Réseau de micro-électrodes (1) selon l'une des revendications précédentes, **caractérisé en ce que** l'une, plusieurs ou la totalité des structures de colonnes (19) est/sont constituée(s) du matériau du substrat.

8. Réseau de micro-électrodes (1) selon l'une des revendications précédentes, **caractérisé en ce que** des ouvertures traversantes (24) sont disposées dans le substrat (4) pour permettre la diffusion de substances pharmacologiques dans le tissu et/ou pour introduire une ou plusieurs électrodes profondes pénétrantes (25) dans le tissu.

9. Réseau de micro-électrodes selon la revendication 8, **caractérisé en ce que** plusieurs ou la totalité des ouvertures traversantes (24) sont disposées selon un motif fixe et prédéterminé qui est réparti sur l'étendue plane du substrat (4).

10. Réseau de micro-électrodes (1) selon la revendication 8 ou 9, **caractérisé en ce qu'**une ou plusieurs électrodes profondes (25) ou au moins un réseau d'électrodes profondes (26) sont introduites dans les ouvertures traversantes (24) pour la mesure électrique simultanée de courants dans des régions cérébrales profondes et/ou pour la stimulation électrique et optique de régions cérébrales profondes.

11. Dispositif comportant un réseau de micro-électrodes selon l'une des revendications précédentes et au moins un dispositif de commande électronique, **caractérisé en ce que** le dispositif de commande électronique est couplé à l'un, à plusieurs ou à la totalité des composants électriques et/ou électroniques du réseau de micro-électrodes (1).

12. Dispositif selon la revendication 11, **caractérisé en ce que** le dispositif de commande électronique est couplé par câble et/ou sans fil à l'un, à plusieurs ou à la totalité des composants électriques et/ou électroniques du réseau de micro-électrodes (1).

13. Dispositif selon l'une des revendications 11 à 12, **caractérisé en ce que** le dispositif de commande électronique est couplé au moins à l'une, à plusieurs ou à la totalité des sources lumineuses électriques (3) et est conçu pour commander la puissance lumineuse de l'une, de plusieurs ou de la totalité des sources lumineuses électriques couplées (3).

14. Dispositif selon la revendication 12, **caractérisé en ce que** le dispositif de commande électronique est conçu pour commander l'émission de lumière de l'une, de plusieurs ou de la totalité des sources lumineuses électriques couplées (3) en fonction de composants capteurs électriques et/ou électroniques du réseau de micro-électrodes (1), qui respectivement un signal de capteur électrique en fonction d'au moins une grandeur physique influencée par la source lumineuse électrique (3) ou par les sources lumineuses électriques (3) et détectée par le composant capteur respectif.
